(19) Europäisches Patentamt
European Patent Office
Office européen des brevets

(11) Veröffentlichungsnummer: **0 116 961**
**A1**

(12) # EUROPÄISCHE PATENTANMELDUNG

(21) Anmeldenummer: 84101613.2

(22) Anmeldetag: 16.02.84

(51) Int. Cl.[3]: C 07 D 239/48
A 01 N 43/54, C 07 D 239/50

(30) Priorität: 18.02.83 DE 3305524

(43) Veröffentlichungstag der Anmeldung:
29.08.84 Patentblatt 84/35

(84) Benannte Vertragsstaaten:
AT BE CH DE FR GB IT LI NL SE

(71) Anmelder: CELAMERCK GmbH & Co. KG
Binger Strasse 173
D-6507 Ingelheim am Rhein(DE)

(72) Erfinder: Mengel, Rudolf, Dr.
Schützenpfad 22
D-6507 Ingelheim(DE)

(72) Erfinder: Schröder, Ludwig, Dr.
Frankenstrasse 7
D-6507 Ingelheim(DE)

(72) Erfinder: Stransky, Werner, Dr.
Im Hippel 24
D-6535 Gau-Algesheim(DE)

(72) Erfinder: Linden, Gerbert, Dr.
Turnierstrasse 44
D-6507 Ingelheim(DE)

(72) Erfinder: Schneider, Gerhart
Schleifmühlenweg 7a
D-6109 Mühltal(DE)

(72) Erfinder: Lust, Sigmund, Dr.
Klappacher Strasse 2f
D-6100 Darmstadt(DE)

(54) **Neue Pyrimidinderivate.**

(57) Die vorliegende Erfindung betrifft neue herbizid wirksame Pyrimidinderivate der allgemeinen Formel

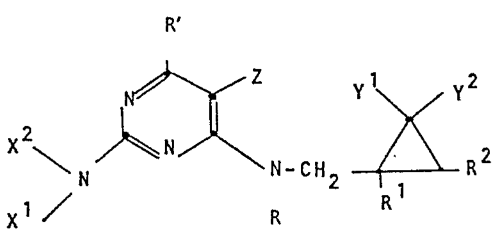

(I),

Verfahren zu ihrer Herstellung sowie ihre Verwendung bei der Bekämpfung unerwünschten Pflanzenwachstums, und bei der Verhütung und Bekämpfung von Mikroorganismenbefall auf Kulturpflanzen.

EP 0 116 961 A1

Croydon Printing Company Ltd.

Die Erfindung betrifft neue Pyrimidinderivate, ihre Herstellung, herbizide und mikrobizide Mittel, die diese Pyrimidine enthalten sowie ihre Verwendung bei der Bekämpfung unerwünschten Pflanzenwachstums und bei der Verhütung und Bekämpfung von Mikroorganismenbefall auf Kulturpflanzen.

Die neuen Verbindungen entsprechen der allgemeinen Formel

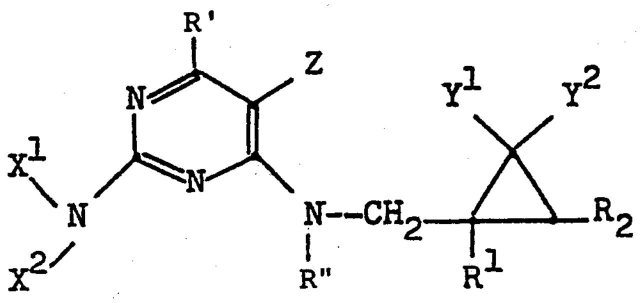

(I),

worin

R' für Wasserstoff, Halogen, einen geradkettigen oder verzweigten $C_1 - C_4$-Alkylrest, Alkoxy, $-SCH_3$ oder $(Alkyl)_2N$;

R" für Wasserstoff, einen geradkettigen oder verzweigten $C_1$-$C_4$-Alkylrest oder für

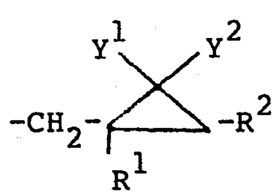

;

$R^1$ und $R^2$, die gleich oder verschieden sein können, für Wasserstoff oder für einen geradkettigen oder verzweigten $C_1$-$C_4$-Alkylrest, der gegebenenfalls durch -O- oder -N- unterbrochen sein kann, insbesondere für Wasserstoff und Methyl;

$X^1$ für Wasserstoff oder für einen gegebenenfalls verzweigten $C_1$-$C_6$-Alkylrest oder $C_2$-$C_5$-Alkenylrest, $R^6$-$SO_2$-, insbesondere für Wasserstoff und Methyl, Ethyl, n-Propyl und n-Butyl;

$X^2$ für Wasserstoff oder für einen gegebenenfalls verzweigten $C_1$-$C_6$-Alkylrest oder $C_2$-$C_5$-Alkenylrest oder für einen gegebenenfalls mit $C_1$-$C_4$-Alkyl und/oder 1 bis 3 Halogenatomen, die gleich oder verschieden sein können, substituierten Phenyl- oder Benzylrest oder für die Reste

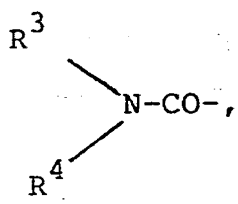 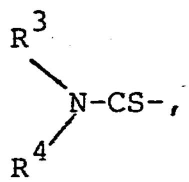 $R^5$-CO-, $R^6$-$SO_2$-, $R^6$-$SO_2$-NH-,

$R^6$-$SO_2$-NH-CO-, $R^7$-O-CO-, 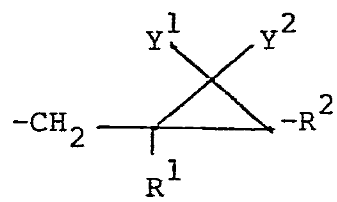

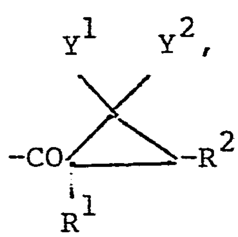 -CH=C$(CN)_2$, -CH=N-CN,

worin

$R^3$ und $R^4$, die gleich oder verschieden sein können, für Wasserstoff oder für einen gegebenenfalls verzweigten $C_1$-$C_4$-Alkylrest,

$R^5$ für Wasserstoff, für einen gegebenenfalls verzweigten $C_1$-$C_6$-Alkylrest, für einen mit ein bis drei Halogenatomen substituierten Methylrest oder für einen gegebenenfalls mit Methyl und/oder ein bis drei Halogenatomen, die gleich oder verschieden sein können, substituierten Phenylrest,

$R^6$ für einen gegebenenfalls verzweigten $C_1$-$C_6$-Alkylrest, für $CF_3$ oder für einen gegebenenfalls mit Methyl, Alkoxy, $COOR^7$ und/oder 1 bis 3 Halogenatomen, die gleich oder verschieden sein können, substituierten Phenyl- oder Benzylrest,

$R^7$ für einen gegebenenfalls verzweigten $C_1$-$C_6$-Alkylrest oder in der

$X^1$ und $X^2$ zusammen den Rest

$(CH_3)_2N\text{-}CH=$

ergeben, jedoch vorzugsweise

$X^2$ für Wasserstoff oder für einen gegebenenfalls verzweigten $C_1$-$C_6$-Alkylrest oder $C_2$-$C_5$-Alkenylrest oder für einen gegebenenfalls mit $C_1$-$C_4$-Alkyl und/oder 1 bis 3 Halogenatomen, die gleich oder verschieden sein können, substituierten Phenyl- oder Benzylrest, Formyl, Acetyl, $CH_3OCO$, $CF_3SO_2$, insbesondere

für Wasserstoff, einen geradkettigen oder verzweigten $C_1$-$C_6$-Alkylrest oder für einen wie oben beschriebenen Phenyl- oder Benzylrest steht;

$Y^1$ für Wasserstoff, Chlor oder Brom,

$Y^2$ für Chlor oder Brom und

Z für Wasserstoff, Halogen oder $-SCH_3$, vorzugsweise für Wasserstoff oder $-SCH_3$, insbesondere für Wasserstoff steht.

Unter Halogen werden Fluor, Chlor und Brom verstanden, insbesondere Chlor; unter Alkyl wird z.B. Methyl, Ethyl, n-Propyl, Isopropyl, n-, sek.- und tert.-Butyl, n-Pentyl, Isoamyl, n-Hexyl usw. und unter Alkoxy z.B. Methyloxy, Ethyloxy, Propyloxy usw. verstanden. Alkenyl bedeutet z.B. Vinyl, Propenyl-(1), Allyl, Butenyl-(1), Butenyl-(2), Butenyl-(3), Pentenyl-(2) usw.

Die neuen Verbindungen werden hergestellt, indem man

a) ein Pyrimidinderivat der Formel

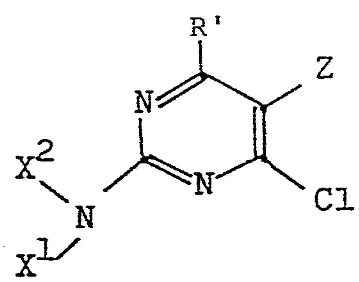

(II),

worin R', $X^1$, $X^2$ und Z die obigen Bedeutungen haben,

 

mit einem Amin der Formel

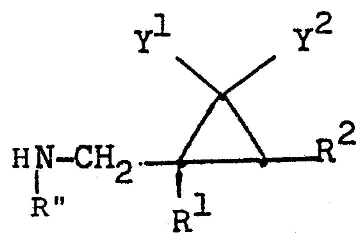 (III),

worin R", $R^1$, $R^2$, $Y^1$ und $Y^2$ die obigen Bedeutungen haben, umsetzt

oder indem man

b) ein Pyrimidinderivat der Formel

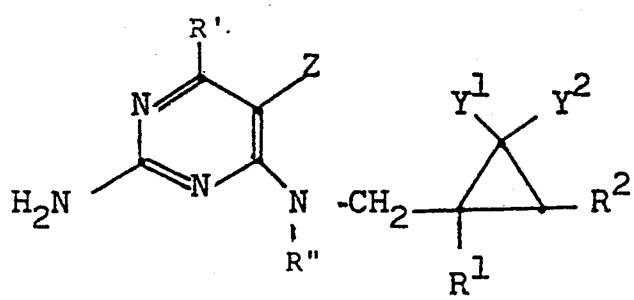 (I a),

worin R', R", $R^1$, $R^2$, $Y^1$, $Y^2$ und Z die obigen Bedeutungen haben, mit geeigneten Acylierungsreagenzien, beispielsweise Essigsäure-ameisensäureanhydrid, Acetylchlorid oder Isocyanaten umsetzt,
und daß man gegebenenfalls nach a) oder b) erhaltene Verbindungen, in denen Z für Wasserstoff steht, mit üblichen Halogenierungsverfahren in solche Verbindungen der Formel I überführt, in denen Z Halogen ist.

Die Umsetzung erfolgt zweckmäßig in einem inerten Lösungsmittel wie Ethanol, Toluol, Methylenchlorid oder Acetonitril, wobei bei der Umsetzung gemäß a) als säurebindendes Mittel beispielsweise Triethylamin verwendet wird. Die Reaktion wird bei Raumtemperatur oder auch erhöhter Temperatur (bis zur Siedetemperatur des Reaktionsgemisches) durchgeführt und das Reaktionsprodukt gewünschtenfalls nach üblichen Verfahren gereinigt. Es empfiehlt sich in Abhängigkeit von der Reaktivität der Ausgangsprodukte gegebenenfalls wasserfrei zu arbeiten.

Der Austausch des Wasserstoffatoms in 5-Stellung des Pyrimidinringes wird vorteilhafterweise bei niedriger Temperatur zwischen -20°C und +30°C in einem niederen, gegebenenfalls halogenierten Kohlenwasserstoff, wie Tetrachlorkohlenstoff oder Methylenchlorid durchgeführt.

Die als Ausgangsprodukte verwendeten Pyrimidinderivate der Formel II sind literaturbekannt oder können nach analogen Verfahren hergestellt werden.

Die Cyclopropylamine der Formel III können mit herkömmlichen Verfahren, z.B. mit Hilfe der Gabrielsynthese hergestellt werden. Cyclopropylamine der Formel III, bei denen $Y^1$ oder $Y^2$ für Wasserstoff steht, werden durch Reduktion der entsprechenden Dihalogenverbindungen erhalten.

Die Verbindungen der Formel I sind herbizid und mikrobizid wirksam.

Man kann sie vor und insbesondere nach dem Auflaufen gegen zahlreiche Unkräuter und Ungräser einsetzen. Zu nennen sind hier besonders:

Amaranthus retroflexus
Abutilon theophrasti
Bidens pilosa
Cassia tora
Centaurea cyanus
Datura stramonium
Desmodium tortuosum
Galium aparine
Ipomoea purpurea
Ipomoea hederacea
Ipomea aconitifolia
Lamium purpurea
Lamium amplexicaule
Lapsana communis
Portulaca oleracea
Sida spinosa
Sinapis arvensis
Stellaria media
Veronica persicaria
Veronica hederaefolia
Xanthium pensylvanicum
Alopecurus myosuroides
Avena fatua
Echinochloa crus-galli
Echinochloa colonum
Eleusine indica
Digitaria sanguinalis
Cynodon dactylon
Leptochloa filiformis
Sorghum halepense
Setaria viridis

Neben der bei hohen Konzentrationen möglichen Anwendung als Totalherbizid erlaubt die gute Selektivität bei niedrigeren Konzentrationen die Unkraut- bzw. Ungrasbekämpfung in zahlreichen Kulturen; beispielsweise in Weizen, Gerste und anderen Getreidearten, in Mais, Sorghum, Reis, Zuckerrohr, Soja, Baumwolle, Zuckerrübe, Kartoffel, Raps u.a.

Für die Anwendung werden die Verbindungen der Formel I in an sich bekannter Weise mit üblichen Hilfs- und oder Trägerstoffen zu gebräuchlichen Formulierungen verarbeitet, z.B. zu Emulsionskonzentraten oder Suspensionspulvern, bei denen der Wirkstoffgehalt zwischen 10 und 95 Gewichtsprozent liegt und die für die Ausbringung mit Wasser bis zur gewünschten Wirkstoffkonzentration verdünnt werden. Jedoch können

auch unverdünnt anwendbare Präparate in Form von Stäuben und Granulaten hergestellt werden. Bei diesen Mitteln liegt der Wirkstoffgehalt zwischen 0,1 und 10 Gewichtsprozent, vorzugsweise zwischen 0,3 und 3 Gewichtsprozent.

Als Trägerstoffe kommen beispielsweise Kaolin, Talkum, Kreide, Aluminiumsilikate, Getreidemehl, Cellulosepulver, Holzmehl u.a. in Frage. Als Dispergatoren finden Kondensationsprodukte des Naphthalins bzw. der Naphthalinsulfonsäuren mit Phenol und Formaldehyd, Salze der Ligninsulfonsäure, Salze sulfatierter Hexa-, Hepta-, Octadecanole u.a. Verwendung. Silicone empfehlen sich als Antischaummittel. Als Lösungsmittel lassen sich Wasser, Alkohole, aromatische Kohlenwasserstoffe, Dimethylsulfoxid, Mineralöle und Pflanzenöle verwenden.

Formulierungsbeispiele
(Angabe der Zusammensetzung in Gewichtsprozent)

1. Emulsionskonzentrat

10 % einer Verbindung der Formel I
40 % Pflanzenöl
10 % Nonylphenolpolyglykolether
37,68 % Cyclohexanon

2. Suspensionspulver

25 % einer Verbindung der Formel I
55 % Kaolin
10 % kolloidale Kieselsäure
9 % Calciumligninsulfonat
1 % Natriumtetrapropylenbenzolsulfonat

3. Suspensionspulver

95 % einer Verbindung der Formel I
4 % Calciumligninsulfonat
1 % Natriumtetrapropylenbenzolsulfonat

4. Stäubemittel

0,3 % einer Verbindung der Formel I
1,0 % Methylcellulose
98,7 % Talkum

Aus den Konzentraten 1 bis 3 werden durch Vermischen mit Wasser Spritzbrühen hergestellt, die im allgemeinen zwischen 0,005 und 0,5 % Wirkstoff enthalten.

Überraschenderweise zeigen die erfindungsgemäßen Verbindungen ein äußerst günstiges Mikrobizid-Spektrum gegen phytopathogene Pilze und Bakterien. Sie besitzen kurative, präventive und systemische Eigenschaften und lassen sich zum Schutz von Kulturpflanzen einsetzen.

Erfindungsgemäße Wirkstoffe lassen sich besonders vorteilhaft gegen folgende Klassen phytopathogener Pilze einsetzen: Ascomyceten, Basidiomyceten, Fungi imperfecti und Phytomyceten.

Folgende Pflanzenarten kommen als behandlungsfähig in Betracht: Getreide, Kern-, Stein- und Beerenobst, Hülsenfrüchte, Ölkulturen, Gurkengewächse, Fasergewächse, Citrusfrüchte, Gemüsearten, Lorbeergewächse, Mais, Tabak, Nüsse, Kaffee, Zuckerrohr, Tee, Weinreben, Hopfen, Bananen und Naturkautschukgewächse sowie Compositen.

Wirkstoffe der allgemeinen Formel I können gleichzeitig oder nacheinander mit weiteren Wirkstoffen auf die zu behandelnde Fläche oder an die Pflanze gegeben werden, wobei diese weiteren Wirkstoffe Präparate, die das Pflanzenwachstum beeinflussen oder Pflanzenschutzmittel sein können.

Für die Anwendung werden die erfindungsgemäßen Verbindungen, gewünschtenfalls auch die Salze, in üblicher Weise mit Hilfs- und/oder Trägerstoffen zu gebräuchlichen Formen von Schädlingsbekämpfungsmitteln verarbeitet, z.B. zu Lösungen, Lösungs- bzw. Emulsionskonzentraten, Suspensionspulvern, Stäuben, Emulsionen. Die Konzentrate werden vor der Anwendung gegebenenfalls mit Wasser verdünnt, so daß Spritzbrühen mit einem Wirkstoffgehalt zwischen etwa 0,005 und 1 Gewichtsprozent erhalten werden. Bei der Anwendung als Low-Volume- oder Ultra-Low-Volume-Formulierung kann der Wirkstoffgehalt auch erheblich höher sein (bis 20 bzw. 50 Gewichtsprozent).

Bevorzugte Applikationsformen sind die Blattapplikation, Bodenapplikation und das Coating der Samenkörner.

Beispiele für erfindungsgemäße Formulierungen:

1. Suspensionspulver

20 Gew.-Teile einer Verbindung nach Formel I
20 Gew.-Teile Kaolin
5 Gew.-Teile Natriumsulfat
2 Gew.-Teile Schlämmkreide
9 Gew.-Teile Calciumligninsulfonat (Dispergiermittel)
1 Gew.-Teil Diisobutylnaphthalin-natriumsulfonat (Netzmittel)
43 Gew.-Teile Kieselkreide

Die Bestandteile werden vermahlen und das Mittel wird für die Anwendung in so viel Wasser suspendiert, daß die Wirkstoffkonzentration etwa 0,005 bis 0,5 Gewichtsprozent beträgt.

2. Emulsionskonzentrat

15 Gew.-Teile einer Verbindung nach Formel I
10 Gew.-Teile Dodecylbenzolsulfonsäure-triäthylaminsalz
75 Gew.-Teile Dimethylformamid

Die Herstellung der Verbindungen der Formel I wird anschließend näher erläutert. Entsprechend den Beispielen können auch die analogen Pyrimidinderivate der Formel I hergestellt werden.

Beispiel I:

2-Amino-4-chlor-6-[[(2,2-dichlorcyclopropyl)methyl]amino]-pyrimidin

38,8 g (0,24 mol) 2-Amino-4,6-dichlorpyrimidin werden in 300 ml Ethanol mit 33,1 g (0,24 mol) 2,2-Dichlorcyclopropylmethylamin und 36 ml (0,26 mol) Triethylamin 6 h unter Rühren zum Rückfluß erhitzt. Das Lösungsmittel wird abdestilliert, der ölige Rückstand mit ca. 300 ml Aceton verrührt und vom Triethylaminhydrochlorid abgesaugt. Man destilliert das Aceton im Vakuum ab und versetzt mit 500 ml Diisopropylether und 100 ml Wasser. Die organische Phase wird nochmals mit 100 ml Wasser gewaschen, über Magnesiumsulfat getrocknet, entfärbt und eingeengt. Nach Verreiben mit Benzin (40° - 60°C) erhält man farblose Kristalle.

Ausbeute: 53,3 g (84,4 % d.Th.)
Smp.: 78° - 79° C

Beispiel II:

2-Formylamino-4-chlor-6-[[(2,2-dichlorcyclopropyl)methyl]-amino]pyrimidin

2,7 g (0,01 mol) 2-Amino-4-chlor-6-[[(2,2-dichlorcyclopropyl)methyl]amino]pyrimidin werden in 300 ml Acetonitril p.A. gelöst vorgelegt und unter Rühren bei Raumtemperatur innerhalb 15 Min. 0,9 g (0,012 mol) Essigsäureameisensäureanhydrid in 5 ml Acetonitril p.A. zugetropft.

Nach ca. 30 h bei Raumtemperatur wird das Lösungsmittel im Vakuum abdestilliert, zweimal mit ca. 30 ml Toluol aufgenommen und jeweils das Toluol wieder abdestilliert. Das Rohprodukt wird säulenchromatographisch gereinigt (150 g Kieselgel, Elutionsmittel: Diisopropylether/ Ethylacetat 9:1).Man erhält weiße Kristalle.

Ausbeute: 1,0 g (33,9 % d.Th.)
Smp. 152°C

Erfindungsgemäß sind die Verbindungen der nachstehenden Tabelle herstellbar.

Tabelle 1

Verbindungen der Formel

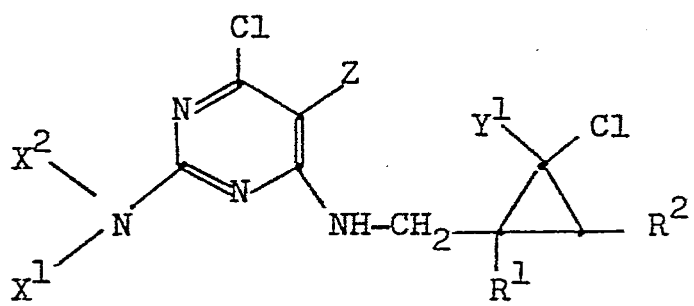

(I b)

| Nr. | $R^1$ | $R^2$ | $X^1$ | $X^2$ | $Y^1$ | Z | Smp. |
|---|---|---|---|---|---|---|---|
| 1 | H | H | H | H | Cl | H | 78-79°C |
| 2 | H | H | H | H | Cl | Cl | 139°C |
| 3 | H | H | H | H | Cl | Br | |
| 4* | H | H | H | H | Cl | $SCH_3$ | |
| 5 | H | H | H | HCO | Cl | H | 152°C |
| 6 | H | H | H | HCO | Cl | Cl | |
| 7 | H | H | H | HCO | Cl | Br | |
| 8* | H | H | H | HCO | Cl | $SCH_3$ | |
| 9 | H | H | H | $CH_3CO$ | Cl | Cl | |
| 10 | H | H | H | $CH_3CO$ | Cl | Br | |
| 11* | H | H | H | $CH_3CO$ | Cl | $SCH_3$ | |
| 12 | H | H | H | $ClCH_2CO$ | Cl | Cl | |

| Nr. | $R^1$ | $R^2$ | $X^1$ | $X^2$ | $Y^1$ | Z | Smp. |
|---|---|---|---|---|---|---|---|
| 13 | H | H | H | $ClCH_2CO$ | Cl | Br | |
| 14* | H | H | H | $ClCH_2CO$ | Cl | $SCH_3$ | |
| 15 | H | H | H | $CF_3CO$ | Cl | Cl | |
| 16 | H | H | H | $CF_3CO$ | Cl | Br | |
| 17* | H | H | H | $CF_3CO$ | Cl | $SCH_3$ | |
| 18 | H | H | H | $(CH_3)_2CHCO$ | Cl | Cl | |
| 19 | H | H | H | $(CH_3)_2CHCO$ | Cl | Br | |
| 20* | H | H | H | $(CH_3)_2CHCO$ | Cl | $SCH_3$ | |
| 21 | H | H | H | $(CH_3)_3CCO$ | Cl | H | Öl |
| 22 | H | H | H | $(CH_3)_3CCO$ | Cl | Cl | |
| 23 | H | H | H | $(CH_3)_3CCO$ | Cl | Br | |
| 24 | H | H | H | $CH_3CH_2CO$ | Cl | H | |
| 25 | H | H | H | $CH_3CH_2CO$ | Cl | Cl | |
| 26 | H | H | H | $CH_3CH_2CO$ | Cl | Br | |
| 27 | H | H | H | $CH_3CH_2CH_2CO$ | Cl | H | |
| 28 | H | H | H | $CH_3CH_2CH_2CO$ | Cl | Cl | |
| 29 | H | H | H | $CH_3CH_2CH_2CO$ | Cl | Br | |
| 30 | H | H | H | n-Bu-CO | Cl | H | |
| 31 | H | H | H | n-Bu-CO | Cl | Cl | |
| 32 | H | H | H | $CH_3$ | Cl | Cl | |
| 33 | H | H | H | $CH_3$ | Cl | H | 105°C |
| 34* | H | H | H | $CH_3$ | Cl | $SCH_3$ | |
| 35 | H | H | H | $C_2H_5$ | Cl | H | 88°C |
| 36 | H | H | H | $(CH_3)_2CH$ | Cl | H | |

| Nr. | $R^1$ | $R^2$ | $X^1$ | $X^2$ | $Y^1$ | Z | Smp. |
|---|---|---|---|---|---|---|---|
| 37 | H | H | $CH_3$ | $CH_3$ | Cl | H | |
| 38 | H | H | $CH_3$ | $CH_3$ | Cl | Cl | |
| 39* | H | H | $CH_3$ | $CH_3$ | Cl | $SCH_3$ | |
| 40 | $CH_3$ | H | H | H | Cl | H | |
| 41 | $CH_3$ | H | H | H | Cl | Cl | |
| 42* | $CH_3$ | H | H | H | Cl | $SCH_3$ | |
| 43 | H | H | H | $CH_3CO$ | Cl | H | |
| 44 | $CH_3$ | H | H | HCO | Cl | H | |
| 45 | $CH_3$ | H | H | $CH_3CO$ | Cl | H | |
| 46 | $CH_3$ | H | H | $ClCH_2CO$ | Cl | H | |
| 47 | $CH_3$ | H | H | $CF_3CO$ | Cl | H | |
| 48 | $CH_3$ | H | H | $(CH_3)_2CHCO$ | Cl | H | |
| 49 | $CH_3$ | H | H | $(CH_3)_3CCO$ | Cl | H | |
| 50 | $CH_3$ | H | H | $CH_3CH_2CO$ | Cl | H | |
| 51 | $CH_3$ | H | H | $CH_3CH_2CH_2CO$ | Cl | H | |
| 52 | $CH_3$ | H | H | n-Bu-CO | Cl | H | |
| 53 | H | $CH_3$ | H | H | Cl | H | |
| 54 | H | $CH_3$ | H | H | Cl | Cl | |
| 55* | H | $CH_3$ | H | H | Cl | $SCH_3$ | |
| 56 | H | $CH_3$ | H | HCO | Cl | H | |
| 57 | H | $CH_3$ | H | $CH_3CO$ | Cl | H | |
| 58 | H | $CH_3$ | H | $CF_3CO$ | Cl | H | |
| 59 | H | $CH_3$ | H | $ClCH_2CO$ | Cl | H | |

| Nr. | $R^1$ | $R^2$ | $X^1$ | $X^2$ | $Y^1$ | Z | Smp. |
|---|---|---|---|---|---|---|---|
| 60 | H | $CH_3$ | H | $CH_3$ | Cl | H | |
| 61 | H | $CH_3$ | $CH_3$ | $CH_3$ | Cl | H | |
| 62 | $CH_3$ | $CH_3$ | H | H | Cl | H | |
| 63 | $CH_3$ | $CH_3$ | H | H | Cl | Cl | |
| 64* | $CH_3$ | $CH_3$ | H | H | Cl | $SCH_3$ | |
| 65 | $CH_3$ | $CH_3$ | H | HCO | Cl | H | |
| 66 | $CH_3$ | $CH_3$ | H | $CH_3CO$ | Cl | H | |
| 67 | $CH_3$ | $CH_3$ | H | $CH_3$ | Cl | H | |
| 68 | $CH_3$ | $CH_3$ | $CH_3$ | $CH_3$ | Cl | H | |
| 69 | $CH_3$ | $CH_3$ | $CH_3$ | $CH_3$ | Cl | Cl | |
| 70* | $CH_3$ | $CH_3$ | $CH_3$ | $CH_3$ | Cl | $SCH_3$ | |
| 71 | H | H | H | 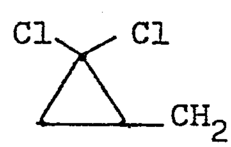 | Cl | H | |
| 72* | H | H | H | 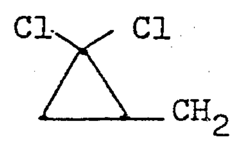 | Cl | $SCH_3$ | 96°C |
| 73 | H | H | H | 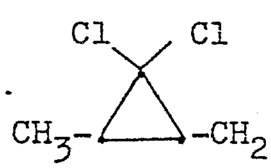 | Cl | H | 98°C |
| 74* | H | $CH_3$ | H | 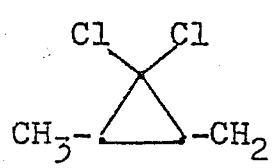 | Cl | $SCH_3$ | |
| 75 | H | H | H | 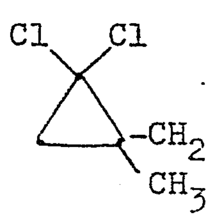 | Cl | H | |

| Nr. | $R^1$ | $R^2$ | $X^1$ | $X^2$ | $Y^1$ | Z | Smp. |
|---|---|---|---|---|---|---|---|
| 76* | $CH_3$ | H | H | 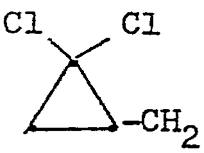 | Cl | $SCH_3$ | |
| 77 | H | $CH_2OCH_3$ | H | H | Cl | H | |
| 78 | H | $CH_2CH_2OCH_3$ | H | H | Cl | H | |
| 79 | H | $CH_2N(CH_3)_2$ | H | H | Cl | H | |
| 80 | H | H | H | 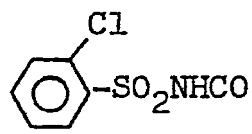 | Cl | H | 215°C |
| 81 | H | H | H | 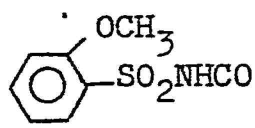 | Cl | H | 174°C |
| 82 | H | H | H | H | H | H | Öl |
| 83* | H | H | H | H | H | $SCH_3$ | |
| 84 | H | H | H | H | H | Cl | |
| 85 | H | H | H | HCO | H | H | |
| 86 | H | H | H | HCO | H | Cl | |
| 87 | H | H | H | $CF_3CO$ | H | H | |
| 88 | H | H | H | $CF_3CO$ | H | Cl | |
| 89 | $CH_3$ | H | H | H | H | H | |
| 90 | $CH_3$ | H | H | H | H | Cl | |
| 91* | $CH_3$ | H | H | H | H | $SCH_3$ | |
| 92 | $CH_3$ | H | H | HCO | H | H | |
| 93 | $CH_3$ | H | H | HCO | H | Cl | |
| 94* | $CH_3$ | H | H | HCO | H | $SCH_3$ | |
| 95 | $CH_3$ | H | H | $CF_3CO$ | H | H | |
| 96 | $CH_3$ | H | H | $CF_3CO$ | H | Cl | |

| Nr. | $R^1$ | $R^2$ | $X^1$ | $X^2$ | $Y^1$ | Z | Smp. |
|---|---|---|---|---|---|---|---|
| 97* | $CH_3$ | H | H | $CF_3CO$ | H | $SCH_3$ | |
| 98 | H | $CH_3$ | H | H | H | H | |
| 99 | H | $CH_3$ | H | HCO | H | Cl | |
| 100 | H | $CH_3$ | H | $CF_3CO$ | H | Cl | |
| 101 | H | H | H | 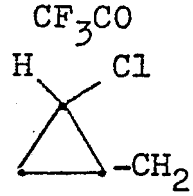 | H | H | |
| 102 | H | H | H | 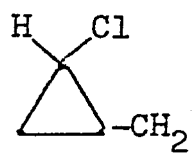 | H | Cl | |
| 103* | H | H | H | 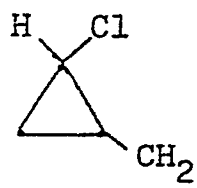 | H | $SCH_3$ | |
| 104 | H | H | H | $CF_3CO$ | Cl | H | Öl |
| 105 | H | H | H | $(CH_3)CHCO$ | Cl | H | |

* Bedingt durch die Herstellung der Ausgangsverbindungen handelt es sich hierbei um ein Gemisch aus 2-gegebenenfalls substituiertem Amino-4-chlor- (siehe obige Formel I b) und 2-Chlor-4- gegebenenfalls substituiertem Aminopyrimidinderivat.

Tabelle II

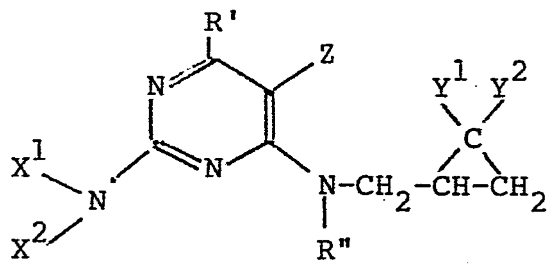

(I c)

| $X^2$ | $X^1$ | R' | Z | R" | $Y^1$ | $Y^2$ | Smp. [°C] |
|---|---|---|---|---|---|---|---|
| 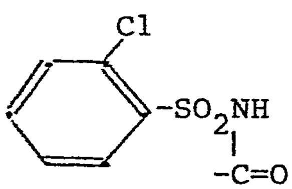 | H | $OCH_3$ | H | H | Cl | Cl | 195 |
| H | H | $OCH_3$ | H | H | Cl | Cl | Öl |
| H | H | $CH_3$ | H | H | Cl | Cl | 118 |
| 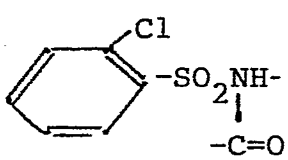 | H | $CH_3$ | H | H | Cl | Cl | amorph 115 |

| $X^2$ | $X^1$ | R' | Z | R" | $Y^1$ | $Y^2$ | Smp. /°C/ |
|---|---|---|---|---|---|---|---|
| H | H | $SCH_3$ | H | H | Cl | Cl | 105 |
| 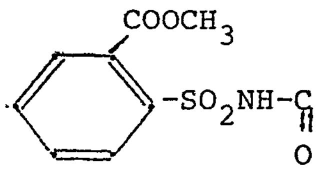 | H | $SCH_3$ | H | H | Cl | Cl | 184 |
| H | H | $(CH_3)_2N$ | H | H | Cl | Cl | 110 |
| 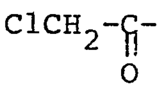 | H | Cl | H | H | Cl | Cl | 116 |
| 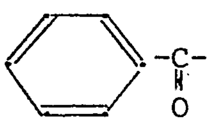 | H | Cl | H | H | Cl | Cl | Öl |
| 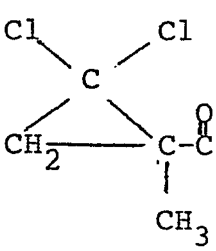 | H | Cl | H | H | Cl | Cl | Öl |
| $CF_3SO_2$ | H | Cl | H | H | Cl | Cl | Öl |

| $X^2$ | $X^1$ | R' | Z | R" | $Y^1$ | $Y^2$ | Smp. [°C] |
|---|---|---|---|---|---|---|---|
| 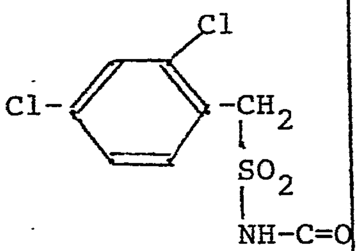 | H | Cl | H | H | Cl | Cl | 103 |
| 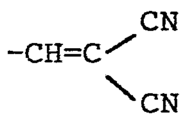 | H | Cl | H | H | Cl | Cl | 174 |
| 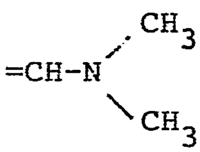 | / | Cl | H | H | Cl | Cl | 186 |
| 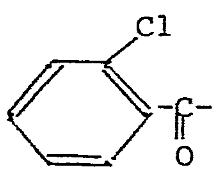 | H | Cl | H | H | Cl | Cl | 156 |
| $CH_3$ | $CH_3$ | Cl | H | H | Cl | Cl | 113 |

| $X^2$ | $X^1$ | R' | Z | R" | $Y^1$ | $Y^2$ | Smp. [°C] |
|---|---|---|---|---|---|---|---|
| H | H | Cl | H | H | Br | Br | 86 |
| $CF_3SO_2$ | $CF_3SO_2$ | Cl | H | H | Cl | Cl | 111 |
| H | H | Cl | H | H | H | H | Öl |
| 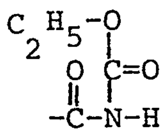 | H | Cl | H | H | Cl | Cl | 224 |
| $CH_3SO_2$ | $CH_3SO_2$ | Cl | H | H | Cl | Cl | 134 |
| 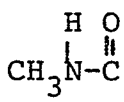 | H | Cl | H | H | Cl | Cl | 210 |
| $CH_3$ | H | Cl | H | H | Cl | H | Öl |
| H | H | Cl | H | 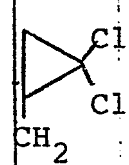 | Cl | Cl | 158 |
| $CH_2=CH-CH_2$ | H | Cl | H | " | Cl | Cl | |
| $CH_3$ | $CH_3$ | Cl | H | H | Cl | H | Öl |
| H | H | Cl | H | $CH_3$ | Cl | Cl | 126 |
| $C_2H_5$ | H | Cl | H | $CH_3$ | Cl | Cl | 89 |
| $CH_2=CH-CH_2$ | H | Cl | H | $CH_3$ | Cl | Cl | |
| 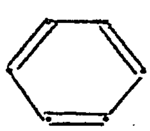 $-CH_2$ | H | Cl | H | H | Cl | Cl | 119 |
| $CH_2=CH-CH_2-CH_2$ | H | Cl | H | H | Cl | Cl | |
| $CH_3-CH_2-CH=CH$ | H | Cl | H | $CH_3$ | Cl | H | |

| $X^2$ | $X^1$ | R' | Z | R" | $Y^1$ | $Y^2$ | Smp. [°C] |
|---|---|---|---|---|---|---|---|
| 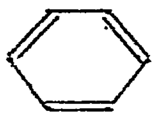 $-CH_2$ | H | Cl | H | H | Cl | H | 106 |
| $C_2H_5$ | H | Cl | H | $C_2H_5$ | Cl | Cl | 109 |
| H | H | Cl | H | H | Cl | Cl | 123 |
| $CH_3-CH=CH-$ | H | Cl | H | H | Cl | H | |
| 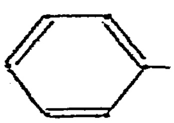 | H | Cl | H | H | Cl | Cl | 101 |
| 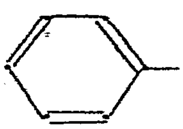 | H | Cl | H | $C_2H_5$ | Cl | Cl | Öl |
| 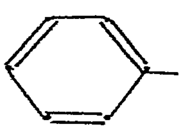 | H | Cl | H | $CH_3$ | Cl | Cl | 123 |
| H | H | $(CH_3)_2N$ | H | $CH_3$ | Cl | Cl | 82 |
| H | H | $OCH_3$ | H | $CH_3$ | Cl | Cl | 91 |
| $CH_3-CH=CH-CH_2$ | H | $CH_3$ | H | H | Cl | Cl | |

Patentansprüche

1) Pyrimidinderivate der Formel

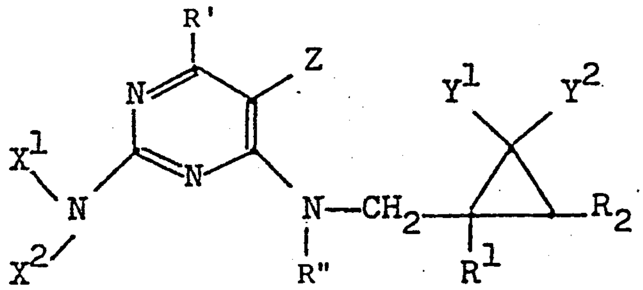

(I),

worin

R' für Wasserstoff, Halogen, einen geradkettigen oder verzweigten $C_1$ - $C_4$-Alkylrest, Alkoxy, $-SCH_3$ oder $(Alkyl)_2N$;

R" für Wasserstoff, einen geradkettigen oder verzweigten $C_1$-$C_4$-Alkylrest oder für

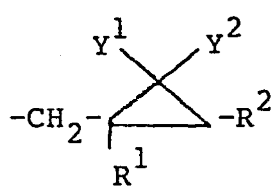

;

$R^1$ und $R^2$, die gleich oder verschieden sein können, für Wasserstoff oder für einen geradkettigen oder verzweigten $C_1$-$C_4$-Alkylrest, der gegebenenfalls durch -O- oder -N- unterbrochen sein kann, insbesondere für Wasserstoff und Methyl;

$X^1$ für Wasserstoff oder für einen gegebenenfalls verzweigten $C_1$-$C_6$-Alkylrest oder $C_2$-$C_5$-Alkenylrest $R_6$-$SO_2$-, insbesondere für Wasserstoff und Methyl, Ethyl, n-Propyl und n-Butyl;

$X^2$ für Wasserstoff oder für einen gegebenenfalls verzweigten $C_1$-$C_6$-Alkylrest oder $C_2$-$C_5$-Alkenylrest oder für einen gegebenenfalls mit $C_1$-$C_4$-Alkyl und/oder 1 bis 3 Halogenatomen, die gleich oder verschieden sein können, substituierten Phenyl- oder Benzylrest oder für die Reste

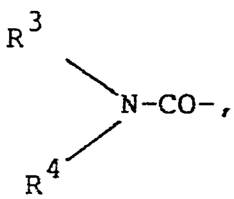, 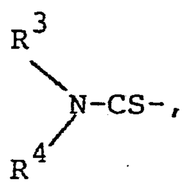, $R^5$-CO-, $R^6$-$SO_2$-, $R^6$-$SO_2$-NH-,

$R^6$-$SO_2$-NH-CO-, $R^7$-O-CO-, 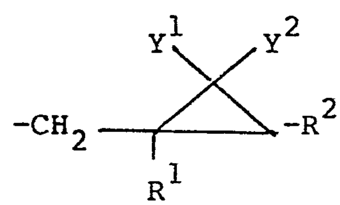

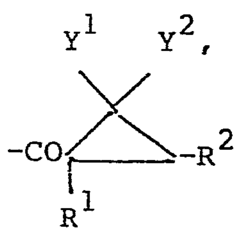, -CH=C(CN)$_2$, -CH=N-CN,

worin

$R^3$ und $R^4$, die gleich oder verschieden sein können, für Wasserstoff oder für einen gegebenenfalls verzweigten $C_1$-$C_4$-Alkylrest,

$R^5$ für Wasserstoff, für einen gegebenenfalls verzweigten $C_1$-$C_6$-Alkylrest, für einen mit ein bis drei Halogenatomen substituierten Methylrest oder für einen gegebenenfalls mit Methyl und/oder ein bis drei Halogenatomen, die gleich oder verschieden sein können, substituierten Phenylrest,

$R^6$ für einen gegebenenfalls verzweigten $C_1$-$C_6$-Alkylrest, für $CF_3$ oder für einen gegebenenfalls mit Methyl, Alkoxy, $COOR^7$ und/oder 1 bis 3 Halogenatomen, die gleich oder verschieden sein können, substituierten Phenyl- oder Benzylrest,

$R^7$ für einen gegebenenfalls verzweigten $C_1$-$C_6$-Alkylrest oder in der

$X^1$ und $X^2$ zusammen den Rest

$$(CH_3)_2N\text{-}CH=$$

ergeben,

$Y^1$ für Wasserstoff, Chlor oder Brom,

$Y^2$ für Chlor oder Brom und

Z für Wasserstoff, Halogen oder $-SCH_3$, vorzugsweise für Wasserstoff oder $-SCH_3$, insbesondere für Wasserstoff steht.

Unter Halogen werden Fluor, Chlor und Brom verstanden, insbesondere Chlor; unter Alkyl wird z.B. Methyl, Ethyl, n-Propyl, Isopropyl, n-, sek,- und tert.-Butyl, n-Pentyl, Isoamyl, n-Hexyl usw. und unter Alkoxy z.B. Methyloxy, Ethyloxy, Propyloxy usw. verstanden. Alkenyl bedeutet z.B. Vinyl, Propenyl-(1), Allyl, Butenyl-(1), Butenyl-(2), Butenyl-(3), Pentenyl-(2) usw.

2) Pyrimidinderivate gemäß Anspruch 1, dadurch gekennzeichnet, daß

$R^1$ und $R^2$, die gleich oder verschieden sein können, für Wasserstoff oder Methyl;

$X^1$ für Wasserstoff;

$X^2$ für Wasserstoff oder für einen gegebenenfalls verzweigten $C_1$-$C_6$-Alkylrest oder $C_2$-$C_5$-Alkenylrest oder für einen gegebenenfalls mit $C_1$-$C_4$-Alkyl und/oder 1 bis 3 Halogenatomen, die gleich oder verschieden sein können, substituierten Phenyl- oder Benzylrest, Formyl, Acetyl, $CH_3OCO$, $CF_3SO_2$ und

Z für Wasserstoff oder $-SCH_3$ steht.

3) Pyrimidinderivate gemäß Anspruch 1, dadurch gekennzeichnet, daß

R' für Halogen

R" für einen geradkettigen oder verzweigten $C_1$-$C_4$-Alkylrest;

$R^1$ und $R^2$, die gleich oder verschieden sein können, für Wasserstoff oder Methyl;

$X^1$ für Wasserstoff;

$X^2$ für Wasserstoff oder für einen gegebenenfalls verzweigten $C_1$-$C_6$-Alkylrest oder $C_2$-$C_5$-Alkenylrest oder für einen gegebenenfalls mit $C_1$-$C_4$-Alkyl und/oder 1 bis 3 Halogenatomen, die gleich oder verschieden sein können, substituierten Phenyl- oder Benzylrest und

Z für Wasserstoff steht.

4) Herbizides Mittel, dadurch gekennzeichnet, daß es als Wirkstoff mindestens ein Pyrimidinderivat der Formel I, Anspruch 1 enthält.

5) Mikrobizides Mittel, dadurch gekennzeichnet, daß es als Wirkstoff mindestens eine Verbindung der Formel I, Anspruch 1 enthält.

6) Verwendung von Verbindungen nach Anspruch 1 bis 3 bei der Bekämpfung unerwünschten Pflanzenwachstums.

7) Verwendung von Verbindungen nach Anspruch 1 bis 3 bei der Verhütung und Bekämpfung von Mikroorganismenbefall auf Kulturpflanzen.

8) Verfahren zur Herstellung von Verbindungen nach Anspruch 1 bis 3, dadurch gekennzeichnet, daß man

a) ein Pyrimidinderivat der Formel

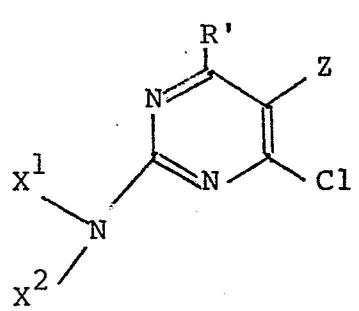

(II),

worin R', $X^1$, $X^2$ und Z die obige Bedeutung haben, mit einem Amin der Formel

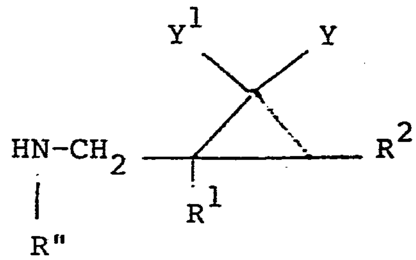

(III)

umsetzt, wobei R", $R^1$, $R^2$, $Y^1$ und $Y^2$ die obige Bedeutung besitzen,

oder daß man

b) ein Pyrimidinderivat der Formel

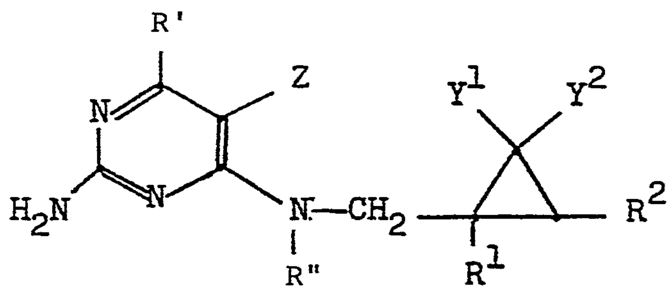

(I a),

worin

R', R", $R^1$, $R^2$, $Y^1$, $Y^2$ und Z die obige Bedeutung haben, mit zur Einführung der Reste $X^1$ und $X^2$ geeigneten Reagenzien umsetzt.

und daß man gegebenenfalls nach a) und b) erhaltene Verbindungen, in denen Z für Wasserstoff steht, mit üblichen Halogenierungsverfahren in solche Verbindungen der Formel I überführt, in denen Z Halogen ist.

9) Pyrimidinderivate gemäß Anspruch 1 bis 3, dadurch gekennzeichnet, daß

$X^1$ für Waserstoff und

$X^2$ für einen gegebenenfalls verzweigten $C_1$-$C_6$-Alkylrest steht.

Europäisches Patentamt

**EUROPÄISCHER RECHERCHENBERICHT**

0116961

Nummer der Anmeldung

EP 84 10 1613

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | EP-A-0 063 509 (PHARMINDUSTRIE) * Seite 22, Beispiel 34; Ansprüche * | 1 | C 07 D 239/48<br>A 01 N 43/54<br>C 07 D 239/50 |
| | --- | | |
| A | EP-A-0 000 681 (UGINE KUHLMANN) * Seiten 1-3; Seite 7, Zeilen 25-32; Seite 8; Ansprüche * | 1,4-9 | |
| | ----- | | **RECHERCHIERTE SACHGEBIETE (Int. Cl. 3)** |
| | | | C 07 D 239/00 |

Der vorliegende Recherchenbericht wurde für alle Patentansprüche erstellt.

| Recherchenort | Abschlußdatum der Recherche | Prüfer |
|---|---|---|
| DEN HAAG | 22-05-1984 | FRANCOIS J.C.L. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82